# EUROPEAN PATENT APPLICATION

(11) **EP 0 705 842 A2**
(43) Date of publication of application: **10.04.1996**
(21) Application number: 95115510.0
(22) Date of filing: 02.10.1995
(51) Int. Cl.: C07K 14/00, C12Q 1/68

(54) **Regulated genes by stimulation of chondrocytes with 1L-1beta**

(30) Priority: 06.10.1994 EP 94115751
(71) Applicant: HOECHST AKTIENGESELLSCHAFT, D-65929 Frankfurt am Main (DE)
(72) Inventor: Bartnik, Eckart, Dr., D-65205 Wiesbaden (DE); Margerie, Daniel, Dipl Biol., D-60320 Frankfurt (DE)

(57) **Abstract**

The present invention refers to the novel use of osteopontin, calnexin and TSG-6 gene product in the diagnosis, prophylaxis or therapy of IL-1β mediated diseases of connective tissues and to novel genes induced or repressed by stimulation of chondrocytes with IL-1β and their use in the diagnosis, prophylaxis or therapy of IL-1β mediated diseases of connective tissues.

## Description

The present invention refers to the novel use of osteopontin and calnexin in the diagnosis, prophylaxis or therapy of IL-1β mediated diseases of connective tissues and to novel genes induced or repressed by stimulation of chondrocytes with IL-1β and their use in the diagnosis, prophylaxis or therapy of IL-1β mediated diseases of connective tissues.

Among the diverse biological effect of interleukin-1 (IL-1), are its actions on the metabolism of many connective tissue cell types including articular chondrocytes. IL-1 inhibits proteoglycan (PG) synthesis by chondrocytes and stimulates production of prostaglandin E₂ and metallo-proteinases capable of degrading matrix macromolecules. From experimental results, and from findings of IL-1, PG fragments and proteolytic enzymes in inflamed joints, it was deduced that IL-1 plays a role in cartilage degradation in osteoarthritis and rheumatoid arthritis (Benton HP & Tyler JA. 1988, Biochem, Biophys. Res. Comm. 154, 421-428; Aydelotte MB et al. Conn. Tiss. Res. 28, 143-159; Wood DD et at., Arthrithis Rheum. 26, 975-983; Lohmander LS et al., Trans Orthrop. Res. Soc. 17, 273). Matrix metalloproteinases are potential candidates for drug interaction at the enzyme level, but relevant molecular targets interfering with earlier processes leading to cartilage degradation are still lacking. Therefore, one objective of the present invention was to identify potential targets for drug modification of IL-1β induced cartilage degradation on the RNA level of human articular chondrocytes from osteoarthritic cartilage.

As an initial attempt to investigate differentially expressed genes in diseased cartilage, total RNA from IL-1β stimulated and unstimulated human chondrocytes was subjected to differential display of mRNA by reverse transcription and polymerase chain reaction (DDRT-PCR). This method can be used to identify and isolate those genes that are differentially expressed in two cell populations (Liang P & Pardee AB 1992, Science 257, 967-971; Liang P et al., AB 1993, Nucl. Acids Res. 21, 3269-3275; Bauer D et al. 1993, Nucl. Acids Res. 21, 4272-4280). The key element is to use a set of oligonucleotide primers, one hybridizing to the polyadenylated tail of mRNAs, the other being arbitrary decamers that anneal at different positions relative to the first primer. mRNA subpopulations defined by these primer pairs are amplified after reverse transcription and resolved on DNA sequencing gels. Band patterns are created, which are characteristic for each RNA population extracted from the cell population under study. For example, 100 different primer combinations should generate a total of approximately 10,000 PCR products for each population, which should represent about the half of all expressed cellular genes. A comparison of the band pattern obtained from two cell populations reveals differentially displayed bands which correspond to differentially expressed genes. Subsequently, differentially displayed bands can be extracted from the gel, reamplified, subcloned and sequenced.

Due to its extreme sensitivity, the appearance of artifactual bands is an inherent problem of the DDRT-PCR method used according to the present application. An additional problem is also the evaluation of complex gene expression patterns. Yet another problem of the present invention is that only minute amounts of RNA are available.

Therefore, it was particularly surprising that the DNA TAU1/1 with the sequences 
TAU1/1(1)
ACATCACCTC ACACATGGAA AGCGAGGAGT TGAATGGTGC ATACAAGGCC ATCCCCGTTT 60
CCCAGGACCT GAACCCGCCT TCTGATTGGG ACAGCCGTGG GAAGGACAGT TATGAAACGA 120
GTCAGCTGGA TGACCAGAGT GCTGAAACCC ACAGCCACAA GCAGTCCAGA TTATATAAGC 180
GGAAA 185
 
and
TAU1/1(2)
CTAAATGCAA AGTGAGAAAT TGTATTTTTT CTCCTTTTAA TTGACCTCAG AAGATGCACT 60
ATCTAATTCA TGAGAAATAC GAAATTTCAG GTGTTTATCT TCTTCCTTAC TTTTGGGG 118
and the DNA TTU2/2 with the sequence 
AACCAGTATT TCAAAACTAT TATCTGGATT CAAGATTAGT GTGTAAAGAT TGTTTTCTTA 60
TCAGTAAAAT AGGTCTTCAG ATCTGCATCT GGCCTCTTAG CATGTTTTTC TTCATAGATA 120
CCCGTTTTGG GGTTTTTGCG TCGGAAGATG AATGGCATTT ATAGTCCTCT CCACATTTAT 180
CTG 183
are 100 % identical to human osteopontin cDNA and 97.2 identical to human calnexin, respectively. This demonstrates that the experimental approach of the present invention worked efficiently, i.e. the use of 100 different primer combinations (25 oligodecamer primers, 4T₁₂MN-primers) generated a total of approximately 10.000 PCR products for each population which represent 53 % of all expressed cellular genes. 123 PCR bands out of 10.000 appeared as differentially expressed bands. 53 of the original 123 PCR bands were reproducibly displayed by comparing the PCR band patterns from two patients; of those 68 % arose from IL-1β stimulated chondrocytes.

It was further found that osteopontin which is a secreted highly acidic phosphoprotein of 32 kd (Denhardt and Guo (1993) FASEB J. 7, 1475-1482) is surprisingly downregulated in IL-1β stimulated human chondrocytes. This means that osteopontin is involved in IL-1β related diseases of connective tissues, in particular osteoarthritis.

Osteoarthritis is characterized as a slowly progressing matrix degeneration with continuing degradation of collagens and proteoglycans and subsequent release of matrix fragments into the synovial fluid. Any disturbance of the normal chondrocyte matrix interactions, for example through a loss of osteopontin, could cause an altered signaling through the integrin alphaᵥbeta₁ and thus changed cellular responses leading to early steps of matrix degradation.

Therefore, one embodiment of the present invention is the use of osteopontin itself or parts thereof, antibodies against it or nucleic acids such as DNA or RNA or parts thereof coding for osteopontin or parts thereof in the diagnoses, prophylaxis or therapy of IL-1β related diseases of connective tissues, in particular osteoarthritis. According to the present application the term "parts" means either at least 8, preferably 12, in particular 15 amino acids in case of proteins or 6-100, preferably 10-40, in particular 12-25 nucleic acids in case of DNA or RNA as hybridization probes. The methods of diagnosing such diseases will be described infra. In addition, quantification on the protein level is possible with osteopontin specific antibodies on Western blots, in immunochemistry, FACS analysis or ELISA based assay systems. The present invention refers also to a diagnosis aid or a pharmaceutical for such use. Osteopontin can be produced for example recombinantly through expression in procayotes, in insect cells in mammalian cells or in mammalian cells using Vaccinia as detailed in Ausubel et al. 1994 [Current protocols in molecular biology, Chapter 16, John Wiley & Sons, Inc]. The cDNA of Osteopontin is e.g. disclosed in Young et al. (1990), Genomics 7, 491 - 502.

Antibodies against osteopontin can be generally produced for example by the method of Neil GA & Urnovitz HB (Trends in Biotechnology, 6, 209-213, 1988) or Köhler G & Milstein C (Nature, 256, 52-53, 1975).

Also calnexin which is an integral membrane protein of 88 kd (Bergeron et al. (1994) TIBS 19, 124-128) is surprisingly downregulated in IL-1β stimulated human chondrocytes compared to unstimulated chondrocytes. This means also that calnexin is involved in IL-1β related diseases of connective tissues, in particular osteoarthritis. In addition, a downregulation of the calnexin synthesis would cause a reduced amount of correctly and completely folded proteoglycans because calnexin is a new type of molecular chaperone that associates with incompletely folded proteins such as proteoglycans. Proteoglycans are highly glycosylated glycoproteins which are of central importance for the maintenance of the cartilage tissue integrity.

Hence, an additional embodiment of the present invention is the use of calnexin itself, or parts thereof antibodies against it or nucleic acids such as DNA or RNA or fragments thereof coding for calnexin or parts thereof in the diagnosis, prophylaxis or therapy of IL-1β related diseases of connective tissues, in particular osteoarthritis. The methods of diagnosing such diseases are already described above. The present invention refers also to a diagnosis aid or a pharmaceutical for such use.

Calnexin can be produced for example recombinantly as described above for osteopontin. The cDNA of Calnexin is e.g. disclosed in Galvin et al. (1992), Proc. Natl. Acad. Sci. USA 89, 8452 - 8456. The production of said antibodies are also generally described above.

### Potential role of identified cDNA fragments in IL-1 mediated cellular processes TSG-6

A homology search in the GenBank and EMBL databases revealed a 99.5 % sequence indentity of fragment TAU7/2(c) with the gene coding for human TSG-6. TSG-6 (TNF stimulated gene 6) was originally isolated by differential cDNA library screening as a TNF induced gene sequence from human fibroblasts (Lee et al., 1990). It was further characterized by Lee et al (1992) as a TNF and IL-1 inducible, secretory, 39 kDa glycoprotein with extensive sequence homology with a region implicated in hyaluronate binding, present in cartilage link protein, proteoglycan core proteins, and the adhesion receptor CD44. With the ability to bind HA and with the most extensive sequence homology to CD44, TSG-6 belongs to the hyaladherin family. Wisniewski et al. (1993) detected high levels of TSG-6 protein in synovial fluids of patients with various forms of arthritis. Six normal control patients did not contain detectable TSG-6 protein in their joint fluid, whereas joint fluids from nine rheumatoid arthritis patients contained high, moderate or low levels of TSG-6. Two patients with osteoarthritis had high levels of TSG-6 in their joint fluids. The apparent local source of TSG-6 in the joints are synoviocytes and chondrocytes (Wisniewski et al., 1993). Lee et al. (1992) speculated that TSG-6 could act as a competitive inhibitor of the interaction between CD44 and its ligand(s) and thus might influence the structural organization of the extracellular matrix of connective tissue, resulting in a destabilization of the proteoglycan aggregates.

Hence, an additional embodiment of the present invention is the use of TSG-6 gene product itself, or parts thereof antibodies against it or nucleic acids such as DNA or RNA or fragments thereof coding for TSG-6 gene product or parts thereof in the diagnosis, prophylaxis or therapy of IL-1β related diseases of connective tissues, in particular osteoarthritis. The methods of diagnosing such diseases are already described above. The present invention refers also to a diagnosis aid or a pharmaceutical for such use.

### Fibronectin

A homology search in the GenBank and EMBL databases revealed a 100 % sequence identity of fragment TTO20/1(c) with the gene coding for human fibronectin.
Fibronectin is a 450 kd glycoprotein with various functions. It acts as an adhesive ligand, as growth or differentiation factor and has chemotactic properties. It is found in the extracellular matrix of most types of cells (Hynes R 1993. Fibronectins, In: Guidebook to the extracellular matrix and adhesion proteins. Editors: Kreis T, Vale R. Oxford University Press. 56-58). An enhanced accumulation of fibronectin and fragments derived from it are found in the synovial fluid and on the inflamed synovial and pannus surfaces in the knee joint of patients with rheumatoid arthritis (Dutu A, Vlaicu-Rus V, Bolosiu HD, Parasca I, Cristea A. 1986. Fibronectin in plasma and synovial fluid of patients with rheumatic diseases. Med. Interne 24, 61-68). Patients with osteoarthritis, as well, have greatly increased levels of fibronectin in their synovial fluid and on cartilage surfaces (Xie D-L, Meyers R, Homandberg GA. 1992. Fibronectin fragments in osteoarthritic synovial fluid. J. Rheumatology 19, 1448-1452). The intraarticular injection of fibronectin fragments causes a severe depletion of cartilage proteoglycans in vivo (Homandberg GA, Meyers R, Williams JM. 1993. Intraarticular injection of fibronectin fragments causes severe depletion of cartilage proteoglycans in vivo. J. of Rheumatology 20, 1378-1382), which is explained by the induced relase of several proteinases, including stromelysin (Xie D-L, Hui F, Meyers R, Homandberg GA. 1994. Cartilage chondrolysis by fibronectin fragments is associated with release of several proteinases: Stromelysin plays a major role in chondrolysis. Arch. Biochem. and Biophysics 311, 205-212). At high concentrations, fibronectin fragments enhance cartilage catabolism through release of cytokines, including IL-1 (Homandberg et al., personal communication).

In respect to these published data, the upregulation of fibronectin by IL-1 can be regarded as a positive feedback regulation, enhancing the self destructive potential of chondrocytes and synoviocytes. With this, fibronectin expression is a direct pharmacological target.

In addition, the sequencing of differentially displayed PCR products discovered also unknown DNA fragments which correspond to differentially expressed genes with or without stimulation of chondrocytes with IL-1β.

Therefore, another embodiment of the present invention is a DNA containing a DNA selected from the group consisting of 
TA08/2(2)
1 CCAAGTTTTT CCAGCAACCC CAAGGGAATA CAGGGAGATC AATGCACCA
51 AAATGGGAAA AGAAAAATAC TTCGATGCAA TGAAACAAAG CCTTTTTCCG
101 TTCAGTTTCC ATAATTCAGT GGTCAGTTTT AAGGCTGCCA CTTGGG

TA016/1(2)
1 GACACGAACA CCACATATTT TTATTGGAGG CCCCATGGCT CCTTGGAAGC
51 CATTTTGGAA CCAAGGGGAC CCACCTTTTT

TA016/2(2)
1 CTAAATATAT TCTCTAACAA GTTAATCTCT TTCAAATCTA TAGATAAAAC
51 TAAAAGGATA AGGAACCAAG GTTTAACCGA CCTAGCCAAT TATGGCAATC
101 ATACTTGCTT TTTAG
 
TA017(C)
1 CATGAAATAT TTCTTGAGGT AATAAGCTTT TACCAAGCTT ATATTTTTGG
51 GCAATTCAGT TACAATGAGA AAAAAACACA CCAAAAGACC AAAAATTTTA

101 AAAACTCACT TTTCTTGCAA TCATAGACAT TTGCATTATT ATAGAACATT
151 CAAACAAGTT AGGTGGATAA TTATTGTCTA TAGATAAATA CGATGCAATT
201 TTTTTAATGT ATGACCGATA CTCCGTATAT ACTTAGATAA CTTATCCAGA
301 AACCTCAACT GTATTGAACA TTGCTGAGAG AAATCAACAA TAATTTTAAC
351 AGATATGATG ACAGNAAAAA TTGATTGCAT ATCTTTTTGC ACTAAAACTT
401 TTATATTTAT TT

TA019(C)
1 AGAGCAGGGG TATTTCNCGG TTCATACCGC CATGGCTTAA GAAGCAAAAG
51 TCATATACCT TAGTAGTGGC AAAGATNGAG GAGATAAAAA AGAGCCTACC
101 CAAGCTGTTG TTGAAGAACA GGTCTTAGAT AAAGAGGAAC CCTTCCAGAA
151 GNACAGAGAC AGGCTAAGGG TGATGCTGAG GAAATGGCTC AGAAGAAACA
201 AGAGATTAA

TAU 1/1(2)
1 CTAAATGCAA AGTGAGAAAT TGTATTTTTT CTCCTTTTAA TTGACCTCAG
51 AAGATGCACT ATCTAATTCA TGAGAAATAC GAAATTTCAG GTGTTTATCT
101 TCTTCCTTAC TTTTGGGG

TAU 1/1(1)
1 ACATCACCTC ACACATGGAA AGCGAGGAGT TGAATGGTGC ATACAAGGCC
51 ATCCCCGTTT CCCAGGACCT GAACCCGCCT TCTGATTGGG ACAGCCGTGG
101 GAAGGACAGT TATGAAACGA GTCAGCTGGA TGACCAGAGT GCTGAAACCC
151 ACAGCCACAA GCAGTCCAGA TTATATAAGC GGAAA

TAU1/2(2)
1 CCGGAATGGG GAGCAAACTA TAAGAACCGG GACCAGTTTC CTCTCTTTGT
51 GCCCTAGTTC CCCCTCCTTT GTATACACCC TCCATCCTGA ATAGACTCTG
101 GTTCTCAGCG TAACACCGAC AACATTCAAT CCTGTAGAGA AACAAATGTT
151 AGCTCAGAAG GACACAGCCT TTGAATCATC AGAGAGTT

TAU 7/1(2)
1 GTTAAGAATA ACTAAATAAA AGTTTTAATT AATTTAGGAA TATAAAAAAC
51 TATTAACATT TAATTTTATA ACTGTATCTG CCAAGCAACT TTAAATATAA
101 TTTATTTACC

TAU 7/1(1)
1 CACGCAATGT GAAATAGGCA CATAGGAAGA ATGGGGAAAC CATCCCCTCA
51 AGCATTTATC CTTTGAGTTA CAAGCAATCC AATTACACTC TTTTAGTTAT
101 TTTTAAATGT ACAGTTAGGT TATTA
 
TAU 7/2(C)
1 CCTTGAAGAT GACCCAGGTT NCTTGGCTGA TTATGTTGAA ATATAGACA
51 GTTACGATGA TGTCCATGGC TTTGTGGGAA GATACTGTGG AGATGAGCTT
101 CCAGATGACA TCATCAGTAC AGGAAATGTC ATGACCTTGA AGTTTCTAAG
151 TGATGCTTCA GTGACAGCTG GAGGTTTCCA AATCAAATAT GTTGCAATGG
201 AT

TAU10(1)
1 GGAGATGACA TTTGCTTTGG GCAGAGGCAG CTAGCCAGGA CACATTTCCA
51 CTATAATTTT ACAAAGTTAA ATTTATAAGC TAGCATTAAG TAAAGTGAAG
101 TTCCAGCTCC CTTGCTAAAA ATAACTAGAG GTAATAATTG GTATTCAGGT
151 AACTCATTTA CATCATAATG TGTTGTGAAA A

TAU12/1(2)
1 TATAAAATAT AAATTATATT ATAAATCATG TATTATTTAT AAAATTATAT
51 TATAAATTTA TAAAAATATA AATTATATTT TAGGCTTAAT GTATAAGGAA
101 TATAAATTAT TAATAAGCAT ATGA

TAU 12/1(1)
1 TGTAATTAAC TGTNCTTGTA GGTCTGTCTT TTATACATGT GTGAGTTTTT
51 CTTTACAATA GATTCCTAGC ATTGGGATTG CTAGGTCAGA TGGTATGCAC
101 ATTTGACATT TTGATTGATA GCACCAGATT GCTTTGTTAA AAAATTTTNN
151 TTTATAGTTT ACATTATCTT TGTACAATAG ATGTTCTCTT TCGAC

TAU 12/2(1)
1 GGGAAGTGAA TTGAAAATAC TTCTTTNTCA ACATAATTTT NGGGTTTTGA
51 AATTGTGTTT GGGTTTTCAG GAAATTGGTG GTAATCTTGT ATTAGACTGAA
101 AAAAAGTGAA TTTTAAAATT CTCAGTGAAG AAGCAAATGA TTTATTTTTC
151 ATAGA

TAU12/3(2)
1 TGTTCTGGTA ACTGTTCTAA TTGTGTCTTT GTTACTTCCA GTGCAACCCT
51 TTCAGGTAAG

TAU12/3(1)
1 CTAAAGAACT TGGTATCTCT ATTAAAGCAC ACGAACCTCC AAGGAAAATA
51 GAGCGATTTA CTCTTCTCAT ATCAGTGCAT ATTTATAAGA AGCACGGAGT
101 CA

TAU13/1(1)
1 AGTCATCAAT TCCTTTTTAT CTGTAATTAC ACATTTGTTT TTATTTCAAA
51 GTAATTATAA GGTGTTATAT TGCATATAAT CAGAAAACTA AATGGAAATA
101 AAATTTTAGT AAGCCCGGCC CCTTTGACCG ATACAGAAAA CTTGA
 
TAU 13/3(2)
1 TATATGGCAG TCTAAAGCAT CAAAGATTTG CATCAACATC TTTCATTTTA
51 GACATCTCCT TGCAATGTAA AATATCATGT ATCAACAACA TCTGGTGCAA
101 ATCCATGAGT CTAACTCGAC ATTCATCTTA GCTCGATTAT TATTCCTTCG
151 TACAGTCGAT GTAAACAATA CAGAAAGAGG ATTATTAAGA ACAGTTT
TAU 13/3(1)
1 ATTCATGAAA TGGTCTATAT GCATGATATT GTAAATTCGG ACTCGAAACC
51 GAAACCAAGG ATTCCGTTAC AAAAATTCCT TAATGCTGAG AATGTTCTCA
101 CGCAAACAAC ATCATGGACA TTAAATTCAA GATATGTGAA TGTTAATTCT
151 GTCAATAAAG TCAACGTAAA GAGTAAAGTT AAAAACAGTT ATATCTNNNC
201 TGTCAATGAT GAGTTTAGTT TAACAGATGA TGAATCAATT CT

TCO 16/1(C)
1 CAAAGTGTTT TTGGTTTTGA GAGAGAGAGA GATTGAGAGA CAGAGAGAGA
51 GAGAGAAACC AAGGGATCAT GATAGTTATA GTCAAATACG AGGTTGGATT
101 ATCTTTTGAA AATGTGTTGG TTCTGTGATA CAAGAGGAAG CTAAGACATA
151 TCGTGGAAAC ATCTCCCCCC TCCACCTTAA TATCAAGAAC AAATTGTGGA
201 ATCTAATGTT AATGAGAAGT AGTTCCCCAC TGTGTCAGAT G

TCO16/2(C)
1 NCATCTGACA CAGTGGGGAA CTACTTCTCA TTAACATTAG ATTCCACAAT
51 TTNNNCTTGA TATTAAGGNN NNNNNGGGAG ATCGTTTCAC GATATCGTCT
101 TAGCTTCCTC TTGTATCACA GAACCAACAC ATTTCAAAAG ATAATCCTTC
151 CTCNNTTTGA CTATAACTAT CATGATCCCT TGGTTCTCTC TCTCTCTCTG
201 CTCTCTCATC TCTCTCTCTC TNAAAACNAA

TCO17(C)
1 ACAGTAGTTA GGAGTTTCTT TACTTACAAA ATCACTGGAA ATGATTAAAT
51 TGCTTTTCCC CCTCCCCAGA GGTGCATTTT TCTTATTTCC ATATAGTAAA
101 GTTGAGCTTT TACAGTGCAT AATGTGACAT TTGGAATGCT TATCAACTGC
151 ATGTAAACAT TAATAACCT

TCO18(C)
1 GTAAATGGTA TTANNNGCTG AAGAAAAAAA ATTTTTCAAG ACCTCTGTTT
51 TTTAACTGAA CTTTATCATT GGCATTGTGG GCTTTGAAGT TGCTGGGATA
101 AATTAATATA ATTAAATAAA AGACTGAATT TAATTGCAAA AAAAAAAAAA
151 AACAATAAGT GTGGTGAT

TCU2/1(1)
1 AAGAAATTAT CCAGTTATTT ACAAGGCCAC TGATATTTTA AACGTCCAAA
51 AGTTTGTTTA AATGGGCTGT TACCGCTGAG AATGATGAGG ATGAGAATGA
101 TGGTTGAAGG TTACATTTTA GGAAATGAAG AAACTTAGAA AATTAATATA
151 AAGACAGTGA TAAATACAAA GAAGATTT
 
TCU2/2(1)
1 CGGGTTAATA TTATCCTCTA GTATAAGTGA ATTACTAGTT TCTCTTTATT
51 TAGACAAACA CACACACACC AGATAATATA AACTTAATAA ATTATCTGTT
101 AATGTAGATT TTATTTAAAA AACTATATTT GAACATTGGT CTTTCTTGGA
151 C

TCU9/1(2)
1 ACATAACAGC TTTTATACAA TGATAAGGAC ATATCATTTG TTTACAAAGA
51 AAGTCTAAAA TTTCAAGAAC ATTCAAAGAG CTAACACAGT AAAGGTCATG
101 CAAGTTCTAG AATAGTGAAT CATGACAGAA CTCATTCATT TTATCCTTTA
151 TCTCC

TCU9/2(2)
1 AAGTATGGGT AGCTAAATTT GCATTAAATT AAAAGTACAT ATAATGCAAC
51 ACCACTCTAC ATCTGTATAC CTACGAATGT ATGTGTACTA CACACCCTTA
101 AAATGTTTTT CAAAGTCTTA ATATATTAGA ACATGTTTTC ATTTTTTCAT
151 GGGATGTTAA TACTATTCTA TGATTAAGAA AATACTAG

TCU10(2)
1 AATACAGTTA TTCTAGCTTT TCATATTCAA TTTGAATGAT CAGAAAAGTA
51 TATTAGTCAC ACAGAATTAA ATATTTTAGA TAGTAAGAAT C

TCU14(2)
1 GAAGTGAAAG TCAGCCCTTT AGCTATTATT TATTGCTTTA TTAGAGCAGA
51 GGGAAGTGAC ACTCATTGCC TTCACAGAGC TCTGCAGAAA TATATGCACA
101 GAGTGGTCAA TGCCAACATC TGAGTAAGTC TTCCAAA

TGO20(2)
1 CAGAACATTA GGATTTATTC CTTGATTAGT TCAAATGATT TCAACAGCTG
51 AATTCCTTGA GATGTGTAAG GCAGGTTGGT CCTTTGGATG GACTGTAGAC
101 TGAAACTTCC TATAACTGTA GTGATATGTA CACAGCTACA TAGCAAAGTG
151 CTTCATTATG AAAATGAAGA A

TGO20(1)
1 CAGTGTGAGA GTCTCATTTC TATGCACAGT GTTTCTCAGG AGGATGGAGC
51 TAGTTAGCTG TCTGTTGTCT GTAGCCCAGC TTGATAATGG AACTATACAG
101 CGAAGAGACA ATCTCTGGCA AGTTTTTGTA GAA

TGU5(C)
1 TTAGAGTAAA ATTCCAAATA AATGCTTTGC TCCAAAATTA CACTAACCAG
51 GCTGGGTCTC TATCATACAT CTTCAATACC CTCAAACCTA GATTGTAAAG
101 TGAAAAAAGT GATTAGCNNT TCCATTTGTT CATTCTGTCA CTCACATTCT
151 TAGGCATTTT AAGGATGAGC AACCTTTGTT TCAGAAAGGG TAAGTAATTA
201 GCCCCCTGGA GGTTACATAG TTATAATTTA GTCTTCAGAA TCCGTTCGAA
 
251 GGGNNNNGTT ACTATTTTTA AGATAATTAG AACCCACCTT GTAGCAATAA
301 AAGTTTTCTT GTCTTTG

TGU8(2)
1 GCGAAAGACT AATCGAACCA TCTAGTAGCT GGTTCCCTCC GAAGTTTCCC
51 TCAGGATA

TGU9/1(2)
1 TTAATGTTTA AATACTACTT TTTTTTCAAG CTTGCCCTAG ATACCAACTG
51 TTTATCTAAC ACACAATTCC AGTGTTGCCA AGCCTCATGC CAATTTGAAG
101 GGAACAGCCA AAACTTATGC ATTCATATAA AAAGAGTCTC TAGGCTCTTA
151 TATCTACATT ATAATTTTT

TGU9/2(2)
1 GGAATAACAT TTTTTTATGA GGGAACCCTT TAAAATGGAT GCACACAGTG
51 GCATTTTCTC CTAGGCTCAA AGCTGAGTAC ACTCCCGTAA TTTTAATAAT
101 ATTTTAGGCA AGTCCTATGA CAATTATACC AACAAGTTTC TTCAACCCCA
151 CCACCACCCC ACCATCTCTA TGC

TGU12(C)
1 GGAGGAAGCT TTATTTGGGA AGAGTGCGGT TCNNTCGGCC CTGATCAGCT
51 CTAGCCTGCC CACCCCATCT CAGCCAGGCG GCTTTACTTC TTCCTGAGCT
101 TCAGGTCTTT CTTCTTCCTG ATTTCCTTGG CCAGCTCCCC AATCAATCTC
151 CAGTACTCAT TGAACTTGAG CTCCGAGNCC TGATTCACAT CCAAGCTCTT
201 CATCTTCT

TGU13/1(C)
1 GGATGTGGTA GTTGATCTTT AATGCCCATT CTAGGTCGGA AAAATCCATG
51 ATCCTAACTT TTAAGAGAAG GTTGGTAACT CTACTTAGGA CTTTTTTTTG
101 TAAGAGGAAT AATGTAGCCT CACCCTTATC TTTCTGGAAA TGTTTAAACC
151 ACTGAAATAT GGAGATCAAA TCCAGCTTAC ACACTGGTAA CTCAAATACT
201 ATTTTTTTTT TAAACTATCT TTTCTAAACT AATCACCCCT CTTGTACATA
251 GAACTTTCTA TCTCAGTGCC AATTCTTAGA GGTTGATGCA AACAGCTCTC
301 CAGAGAGCCT GTGCTATTGT TC

TGU13/2(2)
1 GGGGTGTACA TTTTATTGGA AACCTTAAAT ACTGTTCAGA AAGAATATAT
51 CTTCAATCAA GGTCTTGCCG AGCCTACACA GAAAAATGAA GCTTTTTGGG
101 TTAGGGGCAA GGATATATAC AGTACAGAGG ACAAAGA

TTO16/2(C)
1 ACATTCATTA AAGATGAACT TTCAGCATCT TCACTTGAAG ATCCATCAGA
51 TGATTCTGAG AGGCAGGTCT CCCCCAAAAA TCCACCGCAT GTATTCTTTC
101 GTTTAGAATC TGAAAGCCTC TTTCTTTTCA GGCTTGATGA CTCTTCTAAG
151 GTATTTGTTA TGCCTCTCTT CTGGGTTTTT CGTTTTGCCT TATCAAGTAG
 
201 CTNAAATTCA AACACCATGG CAANAGAAAC TGCTTCTAT

TTO20/1(C)
1 CCACCAGCCT ACTGATCAGC TGGGATGCTC CTGCTGTCAC AGTGAGATAT
51 TACAGGATCA CTTACGGAGA AACAGGAGGA AATAGCCCTG TCCAGGAGTT
101 CACTGTGCCT GGGAGCAAGT CTACAGCTAC CATCAGCGGC CTTAAACCTG
151 GAGTTGATTA TACCATCACT GTGTATGCTG TCACTGGCCG TGGAGACAGC

201 CCCGCAAGCA GCAAGCCAAT TTCCATTAAT TACCGAACAG AAATTGACAA
251 ACCATCCCAG ATGCAAGTGA CCGATGTTCA AGACAACTGT TTTAATAAAA
301 GATTTACATT CCAC

TTO20/2(2)
1 TTGGTACCAC AGTCACAGAA CTGGGGGTCA TTTTCTAGAT GAAACAAACG
51 GAACAAGTTC TCTTCCAACA AAGAAATGTA CTGTAGAAAT TAATTTCCTC
101 CATGAATTTT ATATATTGTG TACAAATATA AGGTATGTAT CTGAATACAA
151 AG

TTU2/1(2)
1 CTAGAACTTC CAAAGGCTGC TTGTCATAGA AGCCATTGCA TCTATAAAGC
51 AACGGCTCCT GTTAAATGGT ATCTCCTTTC TGAGGCTCCT ACTAAAAGTC
101 ATTTGTTACC TAAACCTTAT GTGCCTTAAC AGGCCAATGC TTCTCG

TTU 2/2(C)
1 AACCAGTATT TCAAAACTAT TATCTGGATT CAAGATTAGT GTGTAAAGAT
51 TGTTTTCTTA TCAGTAAAAT AGGTCTTCAG ATCTGCATCT GGCCTCTTAG
101 CATGTTTTTC TTCATAGATA CCCGTTTTGG GGTTTTTGCG TCGGAAGATG
151 AAGTGCAGTT TATAGTCCTC TCCACATTTA TCTG

TTU3(1)
1 GGGTAGAAAG CTGAATAATT TATGAAGGAG AGGGGTCAGG GTTGATTCGG
51 GAGGACCTAT TGGTGCGGGG GCTTTGTATG ATTATGGGCG TTGATTAGTA
101 GTAGTTACTG GTTGAACATT GTTTGTTGGT GTATATATTG TAATTGAGAT
151 TGCTCGGGGG AATAGGTTAT GTGATTAGGA GTAGGGTTAG GATGAGTGGG
201 AAG

TTU 5/1(2)
1 GACAAAAAAA AAAAAACAGG TTTTAAAGCT AGAAATGAAA AGCTACTTAA
51 GTATCTTAAA GGATAAGTTA CTTTATTATA CACTAGAAAC ATACACAATA
101 GCTGAAAACT TAAAAAATCT CACACTGCTG AATGTCTCTG CTGGCTG

TTU5/2(2)
1 GCATCCATTG TACATTGTTT GGTTTGAGGT TACCATGAGG CCTGTAAATA
51 CTATCTTATA ATTTATTATT TCAACCTGAT AAAACTTAAC ACTATTTGCA
101 TAAACAAACA AACGAAAA
 
TTU9/1(1)
1 TAAAATACTG GTTCTTTTAT TCTGCAATAT TTTTAAAAAT CACATTTTCA
51 GCCAGGCGCA GTTTCCCACA CCTGTAATCC GGCACTTTGG GAGGCTGAGA
101 TGGGTGGATC ACAAGGTAGG AGATCAAACA TCCTGGCCAA CATGGTGAAC
151 CTGTTTACT

TTU9/2(2)
1 CAAGTATGGG TAGCTAAATT TGCATTAAAT TAAAAGTACA TATAATGCAA
51 CACCACTCTA CATCTGTATA CCTACGAATG TATGTGTACT ACACACCCTT
101 AAATGTTTCA AAGCTTAATA TATTAGAACA TGTTTTCATT TTCAGGGAG

TTU13(2)
1 GGAAATACAC TAGCATGTGA GCACTGTATA TAAAGCTTGA GGTTAGGAGG
51 TAAAATGAAA GAAATCATTT TTAACTCCTA AGATGT

TTU13(1)
1 TGAATTAAAT GGACTCGTTG AAAGGACAAG GAGATCGGTA ATATCTCTCT
51 AAAGAACTTA TATACTAAAA TCTGTAATTG CCTGTACCAA AAGTTTTAGT
101 CTTCTTTT
or an analog thereof. In accordance with the invention, the term "analog" includes nucleic acids which code for the same protein sequence due to the degeneration of the genetic code, for a protein having conservative amino acids substitutions or deletions that do not eliminate the characteristical feature of this protein, or for a protein having at least about 85 %, and more advantageously at least about 90 %, in particular 95 % amino acid sequence homology.

Other embodiments of the invention provide a vector containing said DNA and a host cell containing said vector.

According to the general knowledge one skilled in the art can also use said nucleic acids of the present invention as a hybridization probe to detect the corresponding genes in an organism or in a sample from on organism or gene mutations thereof.

Therefore, an additional embodiment is a method for isolating a gene which can be induced or repressed by treating chondrocytes that contain this gene by IL-1β containing the steps:
(a) hybridizing a DNA of the present invention under stringent preferably high stringent conditions against DNA or RNA containing said gene, preferably DNA or RNA isolated originally from chondrocytes, in particular human chondrocytes; and
(b) isolating this gene by methods known to a skilled person in the art.

According to the present invention the term "stringent conditions" means hybridization conditions comprising a salt concentration of 4 x SSC (NaCl-citrate buffer) at 62-66°C, and "high stringent conditions" means hybridization conditions comprising a salt concentration of 0,1 x SSC at 68°C. The length of the probes are 6-100, preferably 10-40, in particular 12-25 nucleic acids long.

Yet another embodiment is a process for expressing a gene isolated according to the above-described process containing the steps:
(a) cloning said gene into a suitable expression vector such as the pET series (Studier et al., 1990. Methods in Enzymology 185, 60) for procaryotic expression or the vector CDM8 for mammalian expression (Aruffo and Seed, 1987. Proc. Natl. Acad. Sci. USA 84, 8573) or any other expression system known to one skilled in the art; and
(b) expressing said gene in a suitable host cell such as BL21 series (Studier et al., 1990, supra) for procaryotic expression or COS, cells for mammalian expression (Aruffo and Seed, 1987, supra) or any other expression system known to one skilled in the art;
or a method for producing a protein containing the steps:
(a) culturing a suitable host cell, in particular the above mentioned, containing a vector, in particular an expression vector such as the vectors mentioned above which contains a DNA or a gene of the present invention; and
(b) isolating the expressed protein for example by ultrafiltration, precipitation with chaotropic agents such as urea or column chromatography on e.g. ion exchange chromatography columns as detailed in Ausubel et al. 1994 (supra).

A further embodiment is a diagnostic aid containing a DNA or parts thereof or a gene or parts thereof of the present invention. In particular, quantification of the genes can be achieved on the RNA level by Northern blotting with gene specific probes of the present invention or with gene specific primers in a PCR reaction. Such primers can be synthetically produced using the DNA sequences of the present invention or the sequences of the corresponding genes. Therefore, said nucleic acids are useful for the diagnosis of IL-1β related diseases of connective tissues, in particular osteoarthritis or rheumatoid arthritis.

These nucleic acids can also be used to evaluate the expression of certain genes in small cartilage biopsies and to use these ultimately as disease-specific markers and/or as predictive markers for disease progression of e.g. osteoarthritis. The hybridization conditions can be the same as described above.

Said nucleic acids, however, can also be used for the therapy against the diseases mentioned or for the production of a pharmaceutical.

Therefore, another embodiment of the present invention is also the use of said nucleic acids for the production of a pharmaceutical. For example, as described by Uhlmann & Peyman (Chem. Rev. (1990), 90, 543), Milligan et al. (J. Med. Chem. (1993), 36, 1923) or Stein & Cheng (Science (1993), 261, 1004) such nucleic acids can be used as antisense oligonucleotides or triple helix forming oligonucleotides for the inhibition of gene expression. This is in particular useful if such a disease is caused by the overproduction of a gene product which is directly or indirectly regulated by IL-1β in chondrocytes. The nucleic acids can additionally be modified in order to increase e.g. the stability against nucleases as described e.g. in the literatures mentioned above.

Finally, also the gene product itself produced by a method of the present invention can be used as a pharmaceutical.

In the following the invention is in particular described by the examples and tables:

### Description of the Tables

Table 1 gives an overview on used primers and the complexity of the detected differences in expression.

Table 2 summarizes the result of the sequencing of differentially displayed PCR products after their elution from the sequencing gel, reamplification and subcloning into the pCRII vector. The sequences of TAU1/1(1) and TAU1/1(2) are 100 % identical to human osteopontin cDNA, the sequence of TTU2/2 is 97.2 % identical to human calnexin. bp = base pairs, IL-1 = Interleukin-1 stimulation, Stat. sig. score = statistical significance score: a feature of the BLAST database searching program. This score is determined using an implementation of Karlin's significance formula (Karlin, S. and Altschul, S.F. 1990. Methods for assessing the statistical significance of molecular sequence features by using general scoring schemes. Proc. Natl. Acad. Sci. USA, 87:2264-2268), which calculates the Poisson probability that the observed sequence similarity will occur by chance based on the size and composition of the sequence database as well as on the size and quality of the match. The smaller this number, the more it is likely to see sequence similarities.

### Examples

### Cell culture

Articular cartilage specimen were obtained from two patients (male 65 years old and female 73 years old) undergoing total joint replacement surgery for osteoarthritis. None of these individuals had received treatment by radiation or chemotherapy. Articular cartilage slices were aseptically dissected from both femoral condyles, tibia plateaus and pattellae and subjected to sequential enzymatic digestion with pronase and collagenase as described (Häuselmann HJ et al. 1992, Matrix 12, 116-129) Since it is known that the alginate gel suspension system retains the chondrogenic phenotype [Lohmander LS et al. 1992, Trans. Orthop. Res. Soc. 17, 273.] 4 x 10⁶ chondrocytes were suspended in low viscosity alginate (4 x 10⁶ cells / ml 1,25 % w/v alginate in an isotonic buffered solution) and expressed through a 22gauche needle into 102 mM CaCl solution to form cell entrapping beads which are 1,5-3 mm in diameter and spherical. Alginate beads containing a total number of 2 x 10⁷ cells were fed daily for the first three days with medium F12 / DMEM (50/50) and 10 % fetal calf serum (Sigma) with 25 µg / ml ascorbate and 50 µg / ml gentamycin and were then subdivided into two populations for further three culture days in the presence or absence of 5U / ml rh IL-1β (Genzyme). For cell recovery, alginate beads were finally dissolved into dissolution buffer (55 mM sodiumcitrate, 30 mM EDTA, 0,15 M NACl) and placed at room temperature for 10 min. Viability was checked by eosin-red exclusion and cell number was determined.

### Primer syntheses

Arbitrary oligodecamer primers OPA6 to OPA10, OPA16 to OPA20 and degenerate anchored oligo-dT primers (T₁₂VN) were synthesized using the 392 DNA synthesizer (Applied Biosystems) and purified by denaturing polyacrylamid gel electrophoresis. Some oligodecamer primers, U1 to U15 were purchased from Biometra (Göttingen, FRG).

List of all degenerate 3' oligo dT-primers [T₁₂VN] used for DDRT-PCR:

| Primer | Sequence 5' to 3' |
|---|---|
| T₁₂VA | 5'-T T T T T T T T T T T T V A-3' |
| T₁₂VA | 5'-T T T T T T T T T T T T V T-3' |
| T₁₂VA | 5'-T A T T T T T T T T T T V G-3' |
| T₁₂VA | 5'-T T T T T T T T T T T T V C-3' |
| V = dA, dG, dC; N = dA, dT, dG, dC | |

List of all arbitrary 5' oligodecamer primers used for DDRT-PCR:

| Primer | Sequence 5' to 3' |
|---|---|
| OPA 6 | G G T C C C T G A C |
| OPA 7 | G A A A C G G G T G |
| OPA 8 | G T G A C G G G T G |
| OPA 9 | G C G T A A C G C C |
| OPA 10 | G T G A T C G C A G |
| OPA 16 | A G C C A G C G A A |
| OPA 17 | G A C C G C T T G T |
| OPA 18 | A G G T G A C C G T |
| OPA 19 | C A A A C G T C G G |
| OPA 20 | G T T G C G A T C C |
| U1 | T A C A A C G A G G |
| U2 | T G G A T T G G T C |
| U3 | C T T T C T A C C C |
| U4 | T T T T G G C T C C |
| U5 | G G A A C C A A T C |
| U6 | A A A C T C C G T C |
| U7 | T C G A T A C A G G |
| U8 | T G G T A A A G G G |
| U9 | T C G G T C A T A G |
| U10 | G G T A C T A A G G |
| U11 | T A C C T A A G C G |
| U12 | C T G C T T G A T G |
| U13 | G T T T T C G C A G |
| U14 | G A T C A A G T C C |
| U15 | G A T C C A G T A C |

### RNA isolation and cDNA synthesis

Total RNA from cultured articular chondrocytes was prepared according to a single step method Chomczynski and Sacchi (Chomczynski P & Sacchi N 1987, Anal. Biochem. 162, 156-159) and incubated with 10 U RNasefree DNaseI (Gibco, Eggenstein, FRG) for 30 min at 37°C to remove chromosomal DNA contamination of RNA. After extraction with phenol/choroform (3:1), the supernatant was ethanol precipitated in the presence of 0.3 M NaOAc and RNA was redissolved in DEPC treated water. 0,4 µg total RNA was then reverse transcribed using 200 U M-MLV (Moloney murine leukemia virus) reverse transcriptase (Gibco, Eggenstein, FRG) in a 40 µl reaction volume containing 50 mM Tris-HCl (pH 8,3), 75 mM KCl, 10 mM DTT, 3 mM MgCl₂, dNTP mix (dATP, dTTP, dCTP, dGTP) of 200 µM each, 40 U RNase inhibitor (Boehringer Mannheim, FRG) and 2,5 mM degnerate oligo-dT primer (T₁₂VN) at 37°C for 1 h. Reactions were terminated by heating for 5 min at 95°C.

### PCR amplification

cDNAs were amplified in a DNA thermal cycler (Perkin Elmer, model 480) in 20 µl PCR reactions containing 2.5 µM of the original T₁₂MN-primer used in cDNA synthesis in combination with 0.5 µM arbitrary upstream primer, dNTP mix (dGTP, dCTP, dTTP) of 0,5 µM each, 10 µCi α-[³⁵S]dATP (1000 Ci/mmole, 10 mCi/ml), 10 mM Tris-HCl (pH 8.3) 50 mM KCl, 1,5 mM MgCl, 0,001 % gelatin and 2.5 U AmpliTaq DNA polymerase. Light mineral oil was overlaid and thermal cycling was performed as follows: 94°C for 30 seconds, 40°C for 2 min and 72°C for 30 seconds for 40 cycles followed by 5 min postextension at 72°C. AmliTaq DNA polymerase was purchased from Perkin-Elmer (Weiterstadt, FRG) and α-[³⁵S]dATP was obtained from Amersham-Buchler (Braunschweig, FRG). After addition of 5 µl stop buffer (95 % formamide, 20 mM EDTA, 0,05 % bromphenolblue and 0,05 % xylene cyanol) radiolabeled PCR-fragments were then displayed on 6 % acrylamide/7 M Urea high resolution sequencing gels of 35 x 43 cm in size; dried gels were exposed to X-ray film (Kodak X-OMAT) and exposed for 48 h, which allows rapid identification of differentially expressed genes by side by side comparison of DDRT-PCR band patterns.

### Elution, reamplification and cloning of PCR fragments

PCR fragments identified as differentially expressed bands were cut from acrylamide gels, transferred into Eppendorf tubes and rehydrated for 10 min with 100 µl 10 mM Tris-HCl and 1 mM EDTA at room temperature. After boiling the gel slice for 15 min, the PCR fragment was recovered by ethanol precipitation in the presence of 0.3 M NaAc and 20 µg glycogen as a carrier and redissolved in 10 µl sterile water. 5 µl of this volume was used for reamplification by PCR using appropriate primers and conditions described above except for dNTP concentration of 20 µM and no radioisotope. The reamplified PCR product was visualized by electrophoresis on a 2 % agarose gel and eluted from the gel by ultrafiltration using Ultrafree MC-filters (Millipore). Purified PCR fragments were then cloned into the pCRII-vector (Invitrogen, De Schelp, NL) by the TA cloning method (Kovalic D et al. 1991, Nucleic Acids Research 19, 4640), which allows in-vitro transcription and sequencing from the plasmid.

### Sequencing

Plasmid DNA sequencing of subcloned PCR fragments with either SP6(2) or T7(1) primer was carried out using the chain-termination DNA sequencing method, as described by Sanger et al. (Sanger F et al. 1977, Proc. Natl. Acad. Sci. USA 74, 5463-5467.).

### Sequence analysis

The sequence analysis revealed the sequences of cDNA clones TAO8/2(2), TAO16/1(2), TAO16/2(2), TAO17(c), TAO19(c), TAU1/1(2), TAU1/1(1), TAU1/2(2), TAU7/1(2), TAU7/1(1), TAU7/2(c), TAU10(1), TAU12/1(2), TAU12/1(1), TAU12/2(1), TAU12/3(2), TAU12/3(1), TAU13/1(1), TAU13/3(2), TAU13/3(1), TCO16/1(c), TCO16/2(c), TCO17(c), TCO18(c), TCU2/1(1), TCU2/2(1), TCU9/1(2), TCU9/2(2), TCU10(2), TCU14(1), TCU14(2), TGO20(2), TGO20(1), TGU5(c), TGU8(2), TGU9/1(2), TGU9/2(2), TGU12(c), TGU13/1(c), TGU13/2(2), TTO16/2(c), TTO20/1(c), TTO20/2(2), TTU2/1(2), TTU2/2(c), TTU3(1), TTU5/1(2), TTU5/2(2), TTU9/1(1), TTU9/2(2), TTU13(2), TTU13(1) disclosed on pages 7 to 14 of the specification.

Searching for homology between subcloned PCR fragments and sequences already listed in one of the DNA databases (GenBank or EMBL database) was performed using the FASTA program developed by Pearson and Lipman (Pearson W & Lipman DJ 1988, Proc. Natl. Acad. Sci. USA 85, 2444-2448) included in the GCG software package (Genetics Computer Group, Madison, USA).

### Northern-blot analysis

Cell culture and isolation of RNA was performed exactly as described above. 10 µg of total RNA from both IL-1β stimulated or not stimulated chondrocytes were denatured by heating at 65°C for 10 min in a solution of 50 % formamide, 20 mM MOPS and 2.2 M formaldehyde, separated through a 1 % agarose gel containing 2.2 M formaldehyde in 1 X MOPS and transfered to positively charged nylon membrane (Amersham) by standard blotting procedures [Maniatis et. al 1992]. After UV crosslinking, the blots were prehybridized for 1 h in rapid-hyb-buffer (Amersham) at 65°C. A 330 bp cDNA corresponding to nts 61 to 390 of human osteopontin cDNA (GenBank J04765) and a 340 bp cDNA corresponding to nts 881 to 1220 from human calnexin (GenBank M94859) were radiolabeled for hybridization with α-[³²P]dCTP (3000 Ci/mmol, 10 mCi/ ml) using random nonamer primers (Amersham) up to a specific activity of ∼ 1,5 x 10⁹ dpm / µg DNA. Hybridization was performed for 2,5 h at 65°C in prehybridization solution with 2 ng / ml of labeled probe added. The blot was subsequently washed in 2 X SSC, 0.1 % SDS at 37°C for 15 min (1 X SSC = 0,15 M NaCl, 0.015 M sodium citrate, pH 7,0), followed by two sucessive washes with 1 X SSC , 0.1 % SDS at 65°C for 10 min respectively. If necessary, a final high stringency wash was performed with 0.1 X SSC , 0.1 % SDS at 65°C for 15 min. The blots were then analysed by autoradiography using Kodak X-Omat films at -80°C with intensifying screeens for 2-7 days and intensity of bands was quantified with a phosphorimager (Biorad, model GS-250). All blots were stripped with boiling 0.5 % SDS solution and reprobed with labeled β-actin to demonstrate equal loading of RNA in each lane.

### Northern hybridisations (Results)

Fragment TAU7/2(c), identical to TSG-6, was differentially upregulated in IL-1 stimulated cells. This is in concordance with Lee et al. (1992) which reported for TSG-6 a TNF-α and IL-1 mediated upregulation. Fragment TAU1/1, identical to human osteopontin and fragment TTU2/2, identical to human calnexin, both were weaker expressed in IL-1 stimulated chondrocytes compared with the unstimulated cells. To validate our differential display data, we performed Northern analyses of Osteopontin and calnexin expression in IL-1 stimulated and unstimulated chondrocytes originating from a third patient. Both messages were again downregulated. A phosphorimager quantification revealed an osteopontin downregulation by 79% and a calnexin downregulation by 40% in the RNA population from chondrocytes of the third patient.

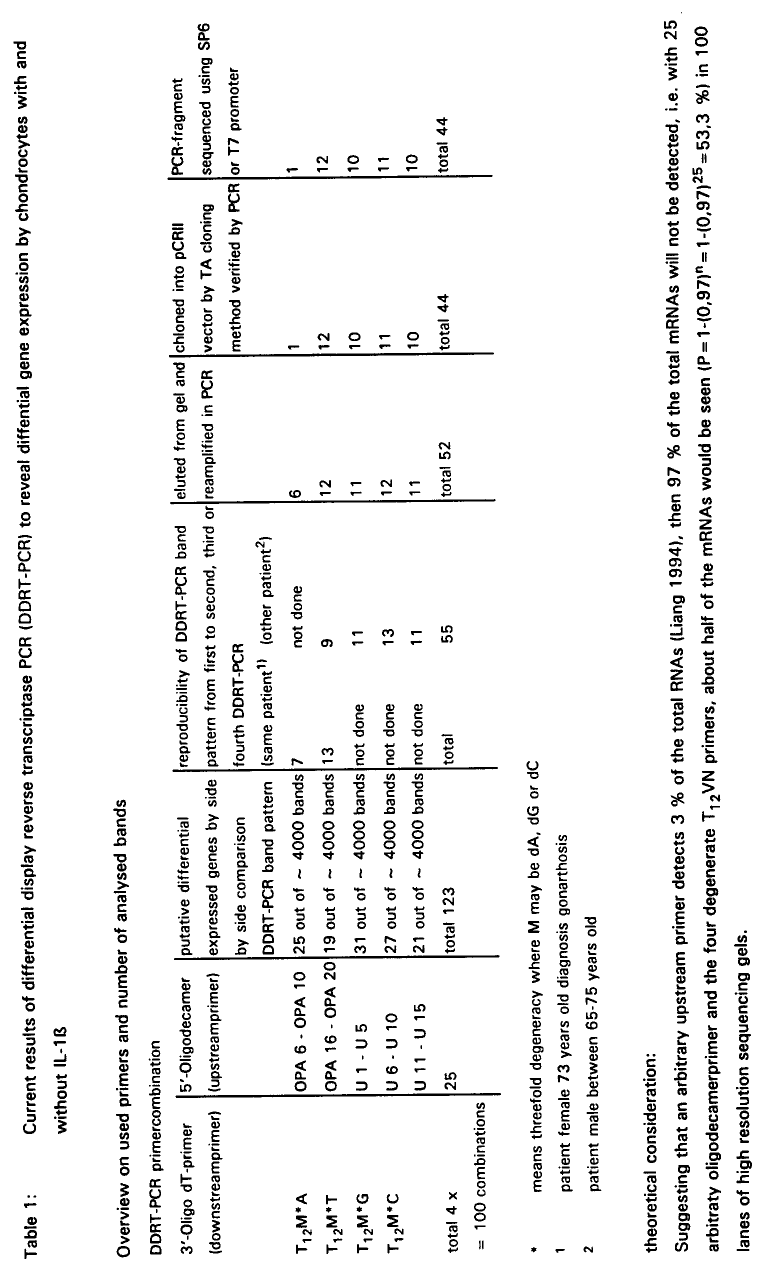

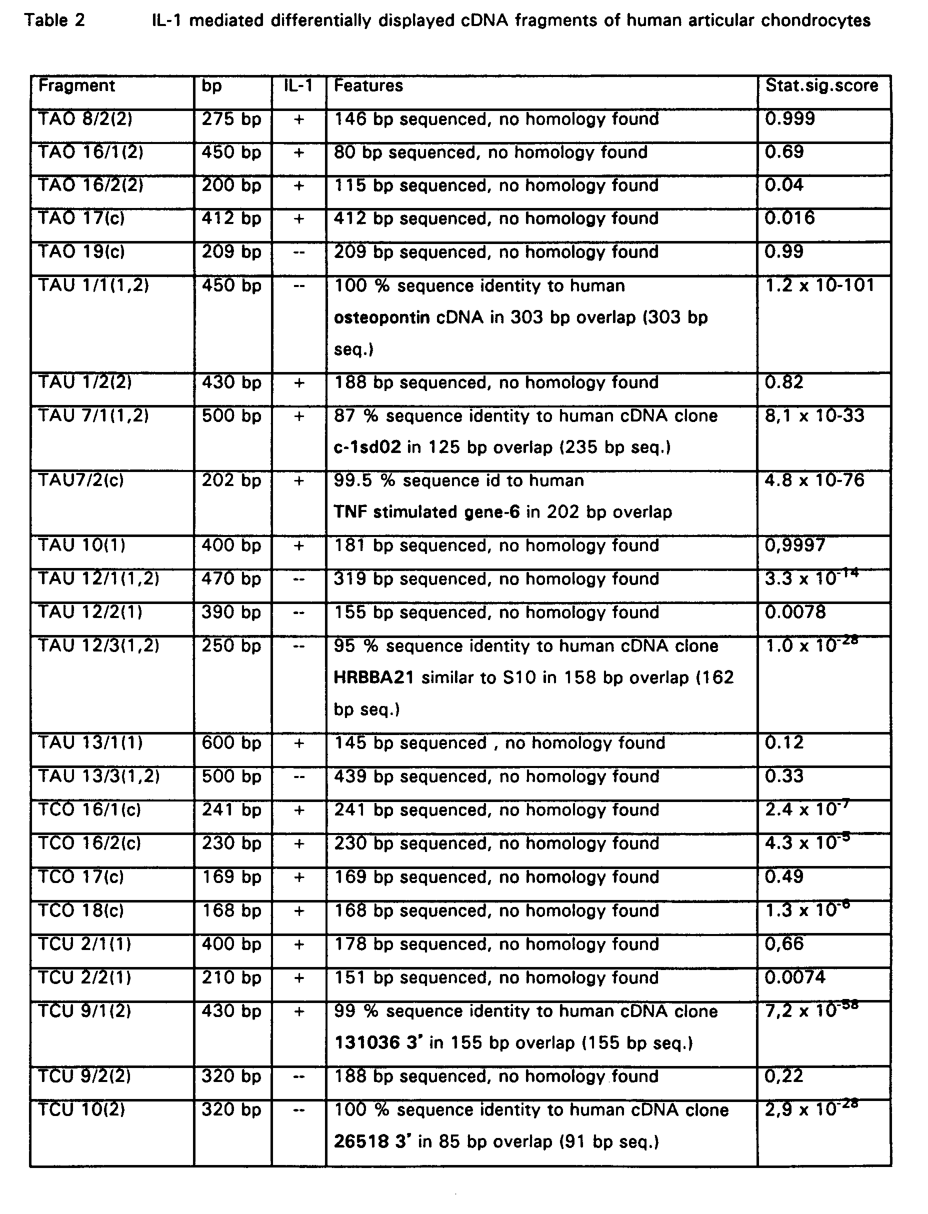

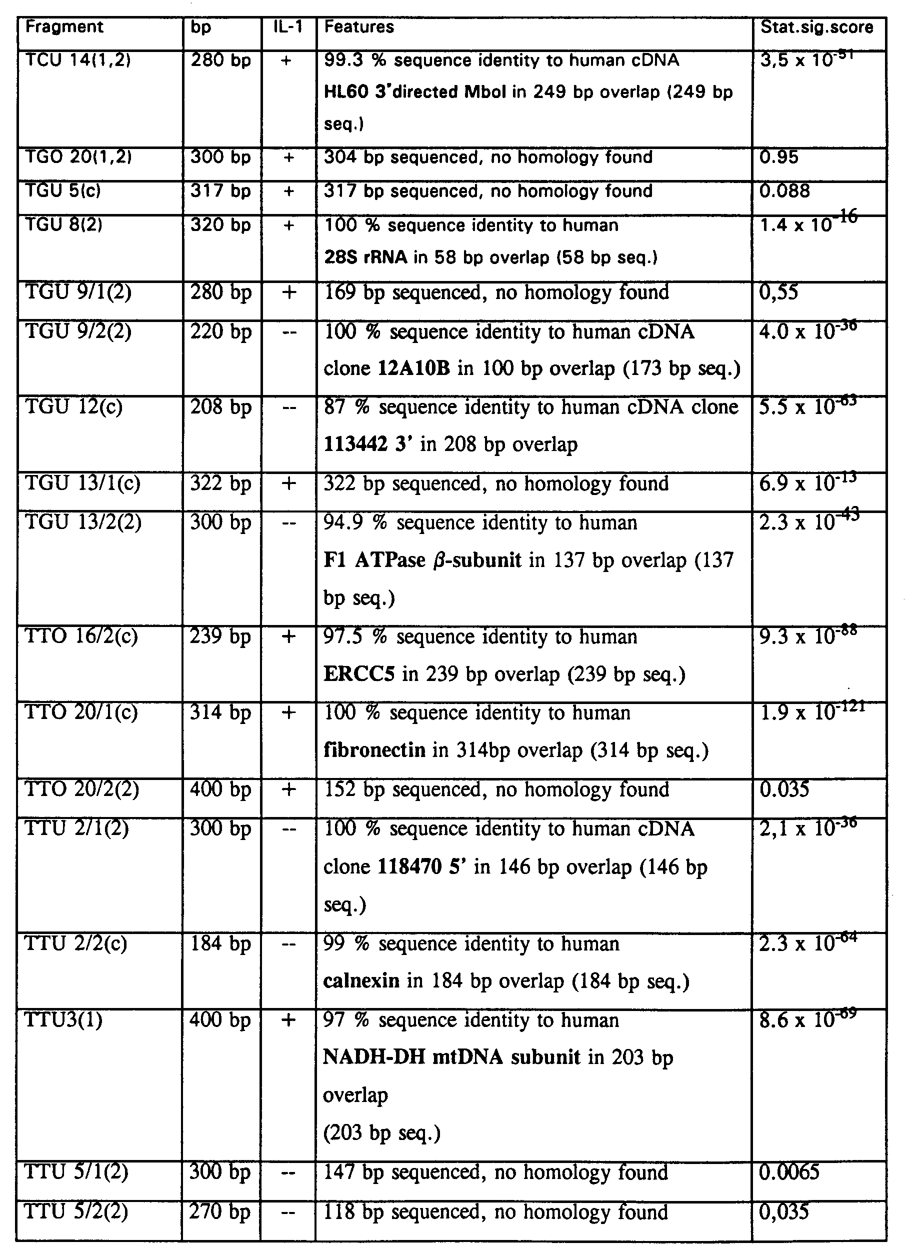

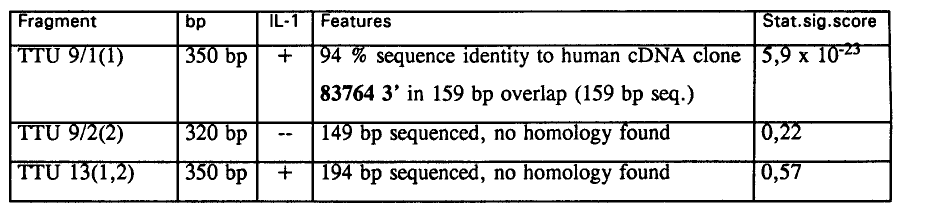

Thus, the 44 identified fragments can be subdivided as follows:
1) 2 fragments with sequence homologies to known human genes with known roles in IL-1 mediated processes:
   - TAU 7/2: identical with human TNF-stimulated gene-6
   - TTO 20/1: identical with human fibronectin
2) 6 fragments with sequence homologies to known human genes, whose function in IL-1 mediated processes can be speculated:
   - TAU 1/1: identical with human osteopontin
   - TGU 8: identical with human 28S ribosomal RNA gene
   - TGU 13/2: identical with human F1 ATPase β-subunit
   - TTO 16/2: identical with human ERCC5
   - TTU 2/2: identical with human calnexin
   - TTU 3: identical with human NADH-DH mtDNA subunit
3) 9 fragments with sequence homologies to human genes, identified in human geneome sequencing projects:
   - TAU 7/1: identical with human cDNA clone c-1sd02
   - TAU 12/3: identical with human cDNA clone HRBBA21
   - TCU 9/1: identical with human cDNA clone 131036 3'
   - TCU 10: identical with human cDNA clone 26518 3'
   - TCU 14: identical with human cDNA clone HL60 3' directed MboI
   - TGU 9/2: identical with human cDNA clone 12A10B
   - TGU 12: identical with human cDNA clone 113442 3'
   - TTU 2/1: identical with human cDNA clone 118470 5'
   - TTU 9/1: identical with human cDNA clone 83764 3'
4) 27 fragments without sequence homologies to known human genes The detection of TSG-6 and fibronectin, both genes known to be upregulated by IL-1, points to the importance of those other cDNA fragments in the light of IL-1 mediated processes. Those genes very likely play roles in degenerate joint diseases, including rheumatoid and osteoarthritis and with this are interesting candidates as markers for clinical studies or as drug targets for pharmacological intervention.

## Claims

1. Use of osteopontin itself or parts thereof, or antibodies against osteopontin or parts thereof or nucleic acids or parts thereof coding for osteopontin or parts thereof in the diagnosis, prophylaxis or therapy of IL-1β a mediated diseases of connective tissues, in particular osteoarthritis.

2. Diagnostic aid for the diagnosis of IL-1β mediated diseases of connective tissues, in particular osteoarthritis, containing osteopontin itself or parts thereof, or antibodies against osteopontin or parts thereof or nucleic acids or parts thereof coding for osteopontin or parts thereof.

3. Pharmaceutical for the prophylaxis or therapy of IL-1β mediated diseases of connective tissues, in particular osteoarthritis, containing osteopontin itself or parts thereof, or antibodies against osteopontin or parts thereof or nucleic acids or parts thereof coding for osteopontin or parts thereof.

4. Use of calnexin itself or parts thereof, or antibodies against calnexin or parts thereof or nucleic acids or parts thereof coding for calnexin or parts thereof in the diagnosis, prophylaxis or therapy of IL-1β mediated diseases of connective tissues, in particular osteoarthritis.

5. Diagnostic aid for the diagnosis of IL-1β mediated diseases of connective tissues, in particular osteoarthritis, containing calnexin itself or parts thereof, or antibodies against calnexin or parts thereof or nucleic acids or parts thereof coding for calnexin or parts thereof.

6. Pharmaceutical for the prophylaxis or therapy of IL-1β mediated diseases of connective tissues, in particular osteoarthritis, containing calnexin itself or parts thereof, or antibodies against calnexin or parts thereof or nucleic acids or parts thereof coding for calnexin or parts thereof.

7. Use of TSG-6 gene product itself or parts thereof, or antibodies against TSG-6 gene product or parts thereof or nucleic acids or parts thereof coding for TSG-6 gene product or parts thereof in the diagnosis, prophylaxis or therapy of IL-1β mediated diseases of connective tissues, in particular osteoarthritis.

8. Diagnostic aid for the diagnosis of IL-1β mediated diseases of connective tissues, in particular osteoarthritis, containing TSG-6 gene product itself or parts thereof, or antibodies against TSG-6 gene product or parts thereof or nucleic acids or parts thereof coding for TSG-6 gene product or parts thereof.

9. Pharmaceutical for the prophylaxis or therapy of IL-1β mediated diseases of connective tissues, in particular osteoarthritis, containing TSG-6 gene product itself or parts thereof, or antibodies against TSG-6 gene product or parts thereof or nucleic acids or parts thereof coding for TSG-6 gene product or parts thereof.

10. DNA containing a DNA selected from the group consisting of 
TAO8/2(2)
1 CCAAGTTTTT CCAGCAACCC CAAGGGAATA CAGGGAGATC AATGCACCCA
51 AAATGGGAAA AGAAAAATAC TTCGATGCAA TGAAACAAAG CCTTTTTCCG
101 TTCAGTTTCC ATAATTCAGT GGTCAGTTTT AAGGCTGCCA CTTGGG

TAO16/1(2)
1 GACACGAACA CCACATATTT TTATTGGAGG CCCCATGGCT CCTTGGAAGC
51 CATTTTGGAA CCAAGGGGAC CCACCTTTTT

TAO16/2(2)
1 CTAAATATAT TCTCTAACAA GTTAATCTCT TTCAAATCTA TAGATAAAAC
51 TAAAAGGATA AGGAACCAAG GTTTAACCGA CCTAGCCAAT TATGGCAATC
101 ATACTTGCTT TTTAG

TAO17(C)
1 CATGAAATAT TTCTTGAGGT AATAAGCTTT TACCAAGCTT ATATTTTTGG
51 GCAATTCAGT TACAATGAGA AAAAAACACA CCAAAAGACC AAAAATTTTA
101 AAAACTCACT TTTCTTGCAA TCATAGACAT TTGCATTATT ATAGAACATT
151 CAAACAAGTT AGGTGGATAA TTATTGTCTA TAGATAAATA CGATGCAATT
201 TTAATAAGAA TTTGAAGAAT GACATTAAAT GCTGTCTGAA GCCTTTGTAT
251 TTTTTAATGT ATGACCGATA CTCCGTATAT ACTTAGATAA CTTATCCAGA
301 AACCTCAACT GTATTGAACA TTGCTGAGAG AAATCAACAA TAATTTTAAC
 
351 AGATATGATG ACAGNAAAAA TTGATTGCAT ATCTTTTTGC ACTAAAACTT
401 TTATATTTAT TT

TAO19(C)
1 AGAGCAGGGG TATTTCNCGG TTCATACCGC CATGGCTTAA GAAGCAAAAG
51 TCATATACCT TAGTAGTGGC AAAGATNGAG GAGATAAAAA AGAGCCTACC
101 CAAGCTGTTG TTGAAGAACA GGTCTTAGAT AAAGAGGAAC CCTTCCAGAA
151 GNACAGAGAC AGGCTAAGGG TGATGCTGAG GAAATGGCTC AGAAGAAACA
201 AGAGATTAA

TAU 1/1(2)
1 CTAAATGCAA AGTGAGAAAT TGTATTTTTT CTCCTTTTAA TTGACCTCAG
51 AAGATGCACT ATCTAATTCA TGAGAAATAC GAAATTTCAG GTGTTTATCT
101 TCTTCCTTAC TTTTGGGG

TAU 1/1(1)
1 ACATCACCTC ACACATGGAA AGCGAGGAGT TGAATGGTGC ATACAAGGCC
51 ATCCCCGTTT CCCAGGACCT GAACCCGCCT TCTGATTGGG ACAGCCGTGG
101 GAAGGACAGT TATGAAACGA GTCAGCTGGA TGACCAGAGT GCTGAAACCC
151 ACAGCCACAA GCAGTCCAGA TTATATAAGC GGAAA

TAU1/2(2)
1 CCGGAATGGG GAGCAAACTA TAAGAACCGG GACCAGTTTC CTCTCTTTGT
51 GCCCTAGTTC CCCCTCCTTT GTATACACCC TCCATCCTGA ATAGACTCTG
101 GTTCTCAGCG TAACACCGAC AACATTCAAT CCTGTAGAGA AACAAATGTT
151 AGCTCAGAAG GACACAGCCT TTGAATCATC AGAGAGTT

TAU 7/1(2)
1 GTTAAGAATA ACTAAATAAA AGTTTTAATT AATTTAGGAA TATAAAAAAC
51 TATTAACATT TAATTTTATA ACTGTATCTG CCAAGCAACT TTAAATATAA
101 TTTATTTACC

TAU 7/1(1)
1 CACGCAATGT GAAATAGGCA CATAGGAAGA ATGGGGAAAC CATCCCCTCA
51 AGCATTTATC CTTTGAGTTA CAAGCAATCC AATTACACTC TTTTAGTTAT
101 TTTTAAATGT ACAGTTAGGT TATTA

TAU 7/2(C)
1 CCTTGAAGAT GACCCAGGTT NCTTGGCTGA TTATGTTGAA ATATATGACA
51 GTTACGATGA TGTCCATGGC TTTGTGGGAA GATACTGTGG AGATGAGCTT
101 CCAGATGACA TCATCAGTAC AGGAAATGTC ATGACCTTGA AGTTTCTAAG
151 TGATGCTTCA GTGACAGCTG GAGGTTTCCA AATCAAATAT GTTGCAATGG
201 AT
 
TAU10(1)
1 GGAGATGACA TTTGCTTTGG GCAGAGGCAG CTAGCCAGGA CACATTTCCA
51 CTATAATTTT ACAAAGTTAA ATTTATAAGC TAGCATTAAG TAAAGTGAAG
101 TTCCAGCTCC CTTGCTAAAA ATAACTAGAG GTAATAATTG GTATTCAGGT
151 AACTCATTTA CATCATAATG TGTTGTGAAA A

TAU12/1(2)
1 TATAAAATAT AAATTATATT ATAAATCATG TATTATTTAT AAAATTATAT
51 TATAAATTTA TAAAAATATA AATTATATTT TAGGCTTAAT GTATAAGGAA
101 TATAAATTAT TAATAAGCAT ATGA

TAU 12/1(1)
1 TGTAATTAAC TGTNCTTGTA GGTCTGTCTT TTATACATGT GTGAGTTTTT
51 CTTTACAATA GATTCCTAGC ATTGGGATTG CTAGGTCAGA TGGTATGCAC
101 ATTTGACATT TTGATTGATA GCACCAGATT GCTTTGTTAA AAAATTTTNN
151 TTTATAGTTT ACATTATCTT TGTACAATAG ATGTTCTCTT TCGAC

TAU 12/2(1)
1 GGGAAGTGAA TTGAAAATAC TTCTTTNTCA ACATAATTTT NGGGTTTTGA
51 AATTGTGTTT GGGTTTTCAG GAAATTGGTG GTAATCTTGT ATTAGCTGAA
101 AAAAAGTGAA TTTTAAAATT CTCAGTGAAG AAGCAAATGA TTTATTTTTC
151 ATAGA

TAU12/3(2)
1 TGTTCTGGTA ACTGTTCTAA TTGTGTCTTT GTTACTTCCA GTGCAACCCT
51 TTCAGGTAAG

TAU12/3(1)
1 CTAAAGAACT TGGTATCTCT ATTAAAGCAC ACGAACCTCC AAGGAAAATA
51 GAGCGATTTA CTCTTCTCAT ATCAGTGCAT ATTTATAAGA AGCACGGAGT
101 CA

TAU13/1(1)
1 AGTCATCAAT TCCTTTTTAT CTGTAATTAC ACATTTGTTT TTATTTCAAA
51 GTAATTATAA GGTGTTATAT TGCATATAAT CAGAAAACTA AATGGAAATA
101 AAATTTTAGT AAGCCCGGCC CCTTTGACCG ATACAGAAAA CTTGA

TAU 13/3(2)
1 TATATGGCAG TCTAAAGCAT CAAAGATTTG CATCAACATC TTTCATTTTA
51 GACATCTCCT TGCAATGTAA AATATCATGT ATCAACAACA TCTGGTGCAA
101 ATCCATGAGT CTAACTCGAC ATTCATCTTA GCTCGATTAT TATTCCTTCG
151 TACAGTCGAT GTAAACAATA CAGAAAGAGG ATTATTAAGA ACAGTTT
 
TAU 13/3(1)
1 ATTCATGAAA TGGTCTATAT GCATGATATT GTAAATTCGG ACTCGAAACC
51 GAAACCAAGG ATTCCGTTAC AAAAATTCCT TAATGCTGAG AATGTTCTCA
101 CGCAAACAAC ATCATGGACA TTAAATTCAA GATATGTGAA TGTTAATTCT
151 GTCAATAAAG TCAACGTAAA GAGTAAAGTT AAAAACAGTT ATATCTNNNC
201 TGTCAATGAT GAGTTTAGTT TAACAGATGA TGAATCAATT CT

TCO 16/1(C)
1 CAAAGTGTTT TTGGTTTTGA GAGAGAGAGA GATTGAGAGA CAGAGAGAGA
51 GAGAGAAACC AAGGGATCAT GATAGTTATA GTCAAATACG AGGTTGGATT
101 ATCTTTTGAA AATGTGTTGG TTCTGTGATA CAAGAGGAAG CTAAGACATA
151 TCGTGGAAAC ATCTCCCCCC TCCACCTTAA TATCAAGAAC AAATTGTGGA
201 ATCTAATGTT AATGAGAAGT AGTTCCCCAC TGTGTCAGAT G

TCO16/2(C)
1 NCATCTGACA CAGTGGGGAA CTACTTCTCA TTAACATTAG ATTCCACAAT
51 TTNNNCTTGA TATTAAGGNN NNNNNGGGAG ATCGTTTCAC GATATCGTCT
101 TAGCTTCCTC TTGTATCACA GAACCAACAC ATTTCAAAAG ATAATCCTTC
151 CTCNNTTTGA CTATAACTAT CATGATCCCT TGGTTCTCTC TCTCTCTCTG
201 CTCTCTCATC TCTCTCTCTC TNAAAACNAA

TCO17(C)
1 ACAGTAGTTA GGAGTTTCTT TACTTACAAA ATCACTGGAA ATGATTAAAT
51 TGCTTTTCCC CCTCCCCAGA GGTGCATTTT TCTTATTTCC ATATAGTAAA
101 GTTGAGCTTT TACAGTGCAT AATGTGACAT TTGGAATGCT TATCAACTGC
151 ATGTAAACAT TAATAACCT

TCO18(C)
1 GTAAATGGTA TTANNNGCTG AAGAAAAAAA ATTTTTCAAG ACCTCTGTTT
51 TTTAACTGAA CTTTATCATT GGCATTGTGG GCTTTGAAGT TGCTGGGATA
101 AATTAATATA ATTAAATAAA AGACTGAATT TAATTGCAAA AAAAAAAAAA
151 AACAATAAGT GTGGTGAT

TCU2/1(1)
1 AAGAAATTAT CCAGTTATTT ACAAGGCCAC TGATATTTTA AACGTCCAAA
51 AGTTTGTTTA AATGGGCTGT TACCGCTGAG AATGATGAGG ATGAGAATGA
101 TGGTTGAAGG TTACATTTTA GGAAATGAAG AAACTTAGAA AATTAATATA
151 AAGACAGTGA TAAATACAAA GAAGATTT

TCU2/2(1)
1 CGGGTTAATA TTATCCTCTA GTATAAGTGA ATTACTAGTT TCTCTTTATT
51 TAGACAAACA CACACACACC AGATAATATA AACTTAATAA ATTATCTGTT
101 AATGTAGATT TTATTTAAAA AACTATATTT GAACATTGGT CTTTCTTGGA
151 C
 
TCU9/1(2)
1 ACATAACAGC TTTTATACAA TGATAAGGAC ATATCATTTG TTTACAAAGA
51 AAGTCTAAAA TTTCAAGAAC ATTCAAAGAG CTAACACAGT AAAGGTCATG
101 CAAGTTCTAG AATAGTGAAT CATGACAGAA CTCATTCATT TTATCCTTTA
151 TCTCC

TCU9/2(2)
1 AAGTATGGGT AGCTAAATTT GCATTAAATT AAAAGTACAT ATAATGCAAC
51 ACCACTCTAC ATCTGTATAC CTACGAATGT ATGTGTACTA CACACCCTTA
101 AAATGTTTTT CAAAGTCTTA ATATATTAGA ACATGTTTTC ATTTTTTCAT
151 GGGATGTTAA TACTATTCTA TGATTAAGAA AATACTAG

TCU10(2)
1 AATACAGTTA TTCTAGCTTT TCATATTCAA TTTGAATGAT CAGAAAAGTA
51 TATTAGTCAC ACAGAATTAA ATATTTTAGA TAGTAAGAAT C

TCU14(1)
1 ATCCTTAGTA AGTGGATTTT GGGGAAAAAA GCACCTGGGC TTCTGGTTCT
51 TTTTGATAAT ATATAAAATT ATTCATTATG AGGTTGCAGT TGTTTGCAAA

TCU14(2)
1 GAAGTGAAAG TCAGCCCTTT AGCTATTATT TATTGCTTTA TTAGAGCAGA
51 GGGAAGTGAC ACTCATTGCC TTCACAGAGC TCTGCAGAAA TATATGCACA
101 GAGTGGTCAA TGCCAACATC TGAGTAAGTC TTCCAAA

TGO20(2)
1 CAGAACATTA GGATTTATTC CTTGATTAGT TCAAATGATT TCAACAGCTG
51 AATTCCTTGA GATGTGTAAG GCAGGTTGGT CCTTTGGATG GACTGTAGAC
101 TGAAACTTCC TATAACTGTA GTGATATGTA CACAGCTACA TAGCAAAGTG
151 CTTCATTATG AAAATGAAGA A

TGO20(1)
1 CAGTGTGAGA GTCTCATTTC TATGCACAGT GTTTCTCAGG AGGATGGAGC
51 TAGTTAGCTG TCTGTTGTCT GTAGCCCAGC TTGATAATGG AACTATACAG
101 CGAAGAGACA ATCTCTGGCA AGTTTTTGTA GAA

TGU5(C)
1 TTAGAGTAAA ATTCCAAATA AATGCTTTGC TCCAAAATTA CACTAACCAG
51 GCTGGGTCTC TATCATACAT CTTCAATACC CTCAAACCTA GATTGTAAAG
101 TGAAAAAAGT GATTAGCNNT TCCATTTGTT CATTCTGTCA CTCACATTCT
151 TAGGCATTTT AAGGATGAGC AACCTTTGTT TCAGAAAGGG TAAGTAATTA
201 GCCCCCTGGA GGTTACATAG TTATAATTTA GTCTTCAGAA TCCGTTCGAA
251 GGGNNNNGTT ACTATTTTTA AGATAATTAG AACCCACCTT GTAGCAATAA
301 AAGTTTTCTT GTCTTTG
 
TGU8(2)
1 GCGAAAGACT AATCGAACCA TCTAGTAGCT GGTTCCCTCC GAAGTTTCCC
51 TCAGGATA

TGU9/1(2)
1 TTAATGTTTA AATACTACTT TTTTTTCAAG CTTGCCCTAG ATACCAACTG
51 TTTATCTAAC ACACAATTCC AGTGTTGCCA AGCCTCATGC CAATTTGAAG
101 GGAACAGCCA AAACTTATGC ATTCATATAA AAAGAGTCTC TAGGCTCTTA
151 TATCTACATT ATAATTTTT

TGU9/2(2)
1 GGAATAACAT TTTTTTATGA GGGAACCCTT TAAAATGGAT GCACACAGTG
51 GCATTTTCTC CTAGGCTCAA AGCTGAGTAC ACTCCCGTAA TTTTAATAAT
101 ATTTTAGGCA AGTCCTATGA CAATTATACC AACAAGTTTC TTCAACCCCA
151 CCACCACCCC ACCATCTCTA TGC

TGU12(C)
1 GGAGGAAGCT TTATTTGGGA AGAGTGCGGT TCNNTCGGCC CTGATCAGCT
51 CTAGCCTGCC CACCCCATCT CAGCCAGGCG GCTTTACTTC TTCCTGAGCT
101 TCAGGTCTTT CTTCTTCCTG ATTTCCTTGG CCAGCTCCCC AATCAATCTC
151 CAGTACTCAT TGAACTTGAG CTCCGAGNCC TGATTCACAT CCAAGCTCTT
201 CATCTTCT

TGU13/1(C)
1 GGATGTGGTA GTTGATCTTT AATGCCCATT CTAGGTCGGA AAAATCCATG
51 ATCCTAACTT TTAAGAGAAG GTTGGTAACT CTACTTAGGA CTTTTTTTTG
101 TAAGAGGAAT AATGTAGCCT CACCCTTATC TTTCTGGAAA TGTTTAAACC
151 ACTGAAATAT GGAGATCAAA TCCAGCTTAC ACACTGGTAA CTCAAATACT
201 ATTTTTTTTT TAAACTATCT TTTCTAAACT AATCACCCCT CTTGTACATA
251 GAACTTTCTA TCTCAGTGCC AATTCTTAGA GGTTGATGCA AACAGCTCTC
301 CAGAGAGCCT GTGCTATTGT TC

TGU13/2(2)
1 GGGGTGTACA TTTTATTGGA AACCTTAAAT ACTGTTCAGA AAGAATATAT
51 CTTCAATCAA GGTCTTGCCG AGCCTACACA GAAAAATGAA GCTTTTTGGG
101 TTAGGGGCAA GGATATATAC AGTACAGAGG ACAAAGA

TTO16/2(C)
1 ACATTCATTA AAGATGAACT TTCAGCATCT TCACTTGAAG ATCCATCAGA
51 TGATTCTGAG AGGCAGGTCT CCCCCAAAAA TCCACCGCAT GTATTCTTTC
101 GTTTAGAATC TGAAAGCCTC TTTCTTTTCA GGCTTGATGA CTCTTCTAAG
151 GTATTTGTTA TGCCTCTCTT CTGGGTTTTT CGTTTTGCCT TATCAAGTAG
201 CTNAAATTCA AACACCATGG CAANAGAAAC TGCTTCTAT
 
TTO20/1(C)
1 CCACCAGCCT ACTGATCAGC TGGGATGCTC CTGCTGTCAC AGTGAGATAT
51 TACAGGATCA CTTACGGAGA AACAGGAGGA AATAGCCCTG TCCAGGAGTT
101 CACTGTGCCT GGGAGCAAGT CTACAGCTAC CATCAGCGGC CTTAAACCTG
151 GAGTTGATTA TACCATCACT GTGTATGCTG TCACTGGCCG TGGAGACAGC
201 CCCGCAAGCA GCAAGCCAAT TTCCATTAAT TACCGAACAG AAATTGACAA
251 ACCATCCCAG ATGCAAGTGA CCGATGTTCA AGACAACTGT TTTAATAAAA
301 GATTTACATT CCAC

TTO20/2(2)
1 TTGGTACCAC AGTCACAGAA CTGGGGGTCA TTTTCTAGAT GAAACAAACG
51 GAACAAGTTC TCTTCCAACA AAGAAATGTA CTGTAGAAAT TAATTTCCTC
101 CATGAATTTT ATATATTGTG TACAAATATA AGGTATGTAT CTGAATACAA
151 AG

TTU2/1(2)
1 CTAGAACTTC CAAAGGCTGC TTGTCATAGA AGCCATTGCA TCTATAAAGC
51 AACGGCTCCT GTTAAATGGT ATCTCCTTTC TGAGGCTCCT ACTAAAAGTC
101 ATTTGTTACC TAAACCTTAT GTGCCTTAAC AGGCCAATGC TTCTCG

TTU 2/2(C)
1 AACCAGTATT TCAAAACTAT TATCTGGATT CAAGATTAGT GTGTAAAGAT
51 TGTTTTCTTA TCAGTAAAAT AGGTCTTCAG ATCTGCATCT GGCCTCTTAG
101 CATGTTTTTC TTCATAGATA CCCGTTTTGG GGTTTTTGCG TCGGAAGATG
151 AAGTGCAGTT TATAGTCCTC TCCACATTTA TCTG

TTU3(1)
1 GGGTAGAAAG CTGAATAATT TATGAAGGAG AGGGGTCAGG GTTGATTCGG
51 GAGGACCTAT TGGTGCGGGG GCTTTGTATG ATTATGGGCG TTGATTAGTA
101 GTAGTTACTG GTTGAACATT GTTTGTTGGT GTATATATTG TAATTGAGAT
151 TGCTCGGGGG AATAGGTTAT GTGATTAGGA GTAGGGTTAG GATGAGTGGG
201 AAG

TTU 5/1(2)
1 GACAAAAAAA AAAAAACAGG TTTTAAAGCT AGAAATGAAA AGCTACTTAA
51 GTATCTTAAA GGATAAGTTA CTTTATTATA CACTAGAAAC ATACACAATA
101 GCTGAAAACT TAAAAAATCT CACACTGCTG AATGTCTCTG CTGGCTG

TTU5/2(2)
1 GCATCCATTG TACATTGTTT GGTTTGAGGT TACCATGAGG CCTGTAAATA
51 CTATCTTATA ATTTATTATT TCAACCTGAT AAAACTTAAC ACTATTTGCA
101 TAAACAAACA AACGAAAA
 
TTU9/1(1)
1 TAAAATACTG GTTCTTTTAT TCTGCAATAT TTTTAAAAAT CACATTTTCA
51 GCCAGGCGCA GTTTCCCACA CCTGTAATCC GGCACTTTGG GAGGCTGAGA
101 TGGGTGGATC ACAAGGTAGG AGATCAAACA TCCTGGCCAA CATGGTGAAC
151 CTGTTTACT

TTU9/2(2)
1 CAAGTATGGG TAGCTAAATT TGCATTAAAT TAAAAGTACA TATAATGCAA
51 CACCACTCTA CATCTGTATA CCTACGAATG TATGTGTACT ACACACCCTT
101 AAATGTTTCA AAGCTTAATA TATTAGAACA TGTTTTCATT TTCAGGGAG

TTU13(2)
1 GGAAATACAC TAGCATGTGA GCACTGTATA TAAAGCTTGA GGTTAGGAGG
51 TAAAATGAAA GAAATCATTT TTAACTCCTA AGATGT

TTU13(1)
1 TGAATTAAAT GGACTCGTTG AAAGGACAAG GAGATCGGTA ATATCTCTCT
51 AAAGAACTTA TATACTAAAA TCTGTAATTG CCTGTACCAA AAGTTTTAGT
101 CTTCTTTT
 or an analog thereof.

11. Vector containing a DNA according to claim 10.

12. Host cell containing a vector according to claim 11.

13. Method for isolating a gene inducable by treating chondrocytes with IL-1β containing the steps:
(a) hybridizing a DNA according to claim 10 under stringent conditions against DNA or RNA containing said gene; and
(b) isolating said gene.

14. A method according to claim 13 wherein said DNA or RNA has been isolated from chondrocytes, particularly human chondrocytes, that were treated with IL-1β.

15. Process for expressing a gene isolated according to claims 13 or 14 containing the steps:
(a) cloning said gene into a suitable expression vector; and
(b) expressing said gene in a suitable host cell.

16. Method for producing a protein containing the steps:
(a) culturing a suitable host cell containing a vector which contains a DNA according to claim 10 or a gene produced by a method according to claim 13 or 14; and
(b) isolating the expressed protein.

17. Diagnostic aid containing a DNA according to claim 10 or parts thereof or a gene isolated according to claim 13 or 14 or parts thereof.

18. Use of a DNA according to claim 10 or parts thereof or a gene isolated according to claim 13 or 14 or parts thereof for the diagnosis, prophylaxis or therapy of IL-1β mediated diseases of connective tissues, in particular osteoarthritis or rheumatoid arthritis.

19. Use of a gene isolated according to claim 13 to 14 for the production of a pharmaceutical.
